# FASCICULE DE BREVET EUROPEEN

(11) **EP 3 995 862 B1**
(45) Date de publication et mention de la délivrance du brevet: **14.08.2024**
(21) Numéro de dépôt: 21207293.8
(22) Date de dépôt: 09.11.2021
(51) Int. Cl.: G01T 1/20, A61B 6/00, G03B 42/04

(54) **STRUCTURE DE DETECTEUR RADIOLOGIQUE**
STRUKTUR EINES RÖNTGENDETEKTORS
STRUCTURE FOR RADIOLOGICAL DETECTOR

(30) Priorité: 09.11.2020 FR 2011467
(43) Date de publication de la demande: 11.05.2022
(73) Titulaire: Trixell, 38430 Moirans (FR)
(72) Inventeur: RIEUVERNET, Pierre, 38430 Moirans (FR)
(74) Mandataire: Atout PI Laplace

(56) Documents cités:
- EP-A1- 0 174 671
- JP-A- 2014 081 364
- US-A1- 2006 038 132
- US-A1- 2008 078 939

## Description

L'invention se situe dans le domaine de l'imagerie. Elle peut être appliquée à tout type d'imageur, notamment les imageurs à rayons X, visibles, infrarouges. L'invention est explicitée ici dans le domaine de l'imagerie médicale par rayons X, ceci à titre d'exemple et sans perte d'applicabilité aux autres domaines d'imagerie. L'invention concerne une cassette radiologique portable et particulièrement une structure innovante de cassette radiologique améliorant la protection de la cassette contre les chutes, impacts d'objets extérieurs, efforts de pression localisés ou répartis, et sollicitations quelconques.

Par le passé, les systèmes radiologiques étaient volumineux et peu mobiles. Il était nécessaire de positionner l'objet par rapport au système pour obtenir l'image désirée. Avec l'apparition de détecteurs à l'état solide, le détecteur est devenu moins volumineux et il a été possible de déplacer le détecteur par rapport à un objet restant fixe. Pour la radiologie médicale, on a réalisé des détecteurs numériques sous forme de cassettes mobiles qu'il devient possible de placer à proximité immédiate d'un patient dont on veut réaliser une image, lorsque l'état de santé du patient empêche son déplacement vers une salle réservée à la radiologie.

Les cassettes radiologiques numériques sont essentiellement constituées d'au moins un scintillateur, dont le rôle est d'émettre une lumière visible sous l'action de rayons X, d'une matrice de photodiodes réalisée sur un substrat généralement en verre, appelée "dalle" dans la suite du document, qui convertit le signal lumineux émis par le scintillateur en charges électriques et d'une ou plusieurs cartes électroniques qui lisent ces charges et les convertissent en une image digitale visible.

Le besoin de souplesse et de réactivité dans l'utilisation de ce type de matériel a conduit les fabricants à proposer des cassettes radiologiques numériques dans un format portable. Ces cassettes portables doivent alors concilier une extrême résistance aux agressions extérieures avec une masse et un format réduits. En effet, ces cassettes portables peuvent être exposées lors de leur manipulation, et tout au long de leur vie, à des chutes, impacts d'objets extérieurs, efforts de pression localisés ou répartis, et des sollicitations de flexion lorsque le poids d'un patient s'exerce sur un détecteur qui n'est pas supporté de façon homogène. Pour cela, la structure mécanique des détecteurs doit assurer une protection maximale des éléments fragiles que sont le scintillateur, la dalle et la carte électronique.

Dans la conception traditionnelle des cassettes radiologiques portables, le scintillateur est associé à la dalle pour constituer un sous-ensemble appelé panneau. Ce panneau est maintenu contre une embase qui lui apporte de la rigidité ainsi qu'un support mécanique. Enfin ce panneau est équipé d'une ou plusieurs cartes électroniques avant d'être inséré dans un boîtier. Pour limiter la masse totale de la cassette portable et assurer une faible absorption des rayons X la traversant, l'épaisseur de ce boîtier, et particulièrement l'épaisseur de la face avant du boîtier faisant face à la source de rayon X est généralement limitée.

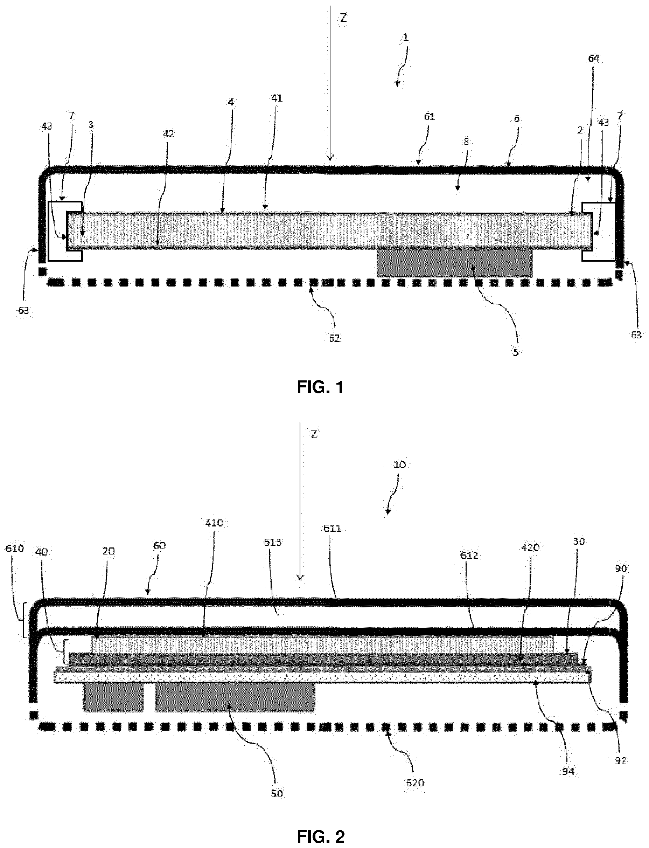

La figure 1 représente une vue en coupe d'une structure de cassette radiologique portable connue de l'art antérieur. Classiquement, une cassette radiologique portable 1 comprend :
- un scintillateur 2 apte à convertir un rayon X incident en un signal lumineux,
- une dalle 3 photosensible apte à convertir le signal lumineux émis par le scintillateur 2, après conversion du rayon X, en une charge électrique. Le scintillateur 2 et la dalle 3 photosensible forment alors un panneau 4. Le panneau 4 comprend une face avant 41 destinée à recevoir le rayon X incident selon une direction d'incidence Z des rayons X, une face arrière 42 opposée à la face avant 41 et deux bords latéraux 43 (dans la vue en coupe),
- une carte électronique 5 qui assure la conversion de la charge électrique en une image digitale,
- un boîtier 6 de protection mécanique, dans lequel sont disposés le panneau 4 et la carte électronique 5, qui comporte une face supérieure 61 contre laquelle le rayon X incident est projeté, un fond 62 opposé à la face supérieure 61 et deux faces latérales 63 (dans la vue en coupe).

La cassette radiologique portable 1 comprend également deux supports de fixation 7 positionnés à l'intérieur du boîtier 6, chaque support de fixation 7 étant placé contre une face latérale 63 du boîtier. Les deux supports de fixation 7 forment une sorte d'embase pour le panneau 4.

Le panneau est maintenu au niveau de ses bords latéraux 43 contre les faces latérales 63 du boîtier 6 à l'aide des supports de fixation 7. Il existe donc au moins une cavité 64 à l'intérieure du boîtier 6 qui définit un espace vide entre la face avant 41 du panneau 4 et la face supérieure 61 du boîtier 6. Il peut aussi, de manière facultative, exister un second espace vide entre la face arrière 42 du panneau 4 et le fond 62 du boîtier 6 remplit partiellement par la carte électronique 5.

Une mousse souple 8 est alors insérée dans la cavité 64 afin de la remplir intégralement et d'être en contact physique avec face avant 41 du panneau 4 suivant un côté et avec la face supérieure 61 du boîtier 6 suivant un autre côté. La norme ISO 4090 étant restrictive du point de vue des dimensions de la cassette portable, la mousse souple 8 doit être extrêmement malléable. Cette mousse souple 8 fournit une isolation contre les chocs dans la direction d'incidence Z des rayons X.

Ainsi, l'embase formée par les supports de fixation 7 apporte la rigidité et évite une trop grande déformation du panneau 4 lors d'éventuelles chutes de la cassette portable ou en situation de flexion, et la mousse souple 8 du boîtier 6 permet de protéger le panneau 4 des impacts. Enfin, la rigidité combinée du boîtier 6 (accompagné de la mousse souple 8) et de l'embase évitent les fortes dégradations dans des cas de pression sur la cassette.

Néanmoins, des contraintes d'épaisseur sur le boîtier, et notamment le respect de la norme ISO 4090 qui limite cette dimension à 15 millimètres, empêchent de donner à chacune de ces pièces, à savoir l'embase, le boîtier 6 et la mousse souple 8, l'épaisseur qui serait souhaitable pour accomplir pleinement leur fonction.

De ce fait, les cassettes portables 1 selon l'état de l'art représenté en figure 1 ne s'avèrent pas aussi robustes que souhaité.

D'autres structures comme présentées dans le document US 2006038132 ont également été envisagées. De fait le document US2006038132 décrit une cassette radiologique portable comprenant : un scintillateur, une dalle photosensible, le scintillateur et la dalle photosensible formant un panneau, le panneau ayant une face avant destinée à recevoir le rayon X incident et une face arrière opposée à la face avant, une carte électronique, un boîtier de protection mécanique dans lequel sont disposés le panneau et la carte électronique, comportant une face supérieure et une face inférieure; la face supérieure du boîtier de protection mécanique comprenant: une première couche de matériau rigide, une deuxième couche de matériau rigide, la deuxième couche de matériau rigide étant en contact avec la face avant du panneau, et une couche de matériau alvéolaire disposée entre la première et la deuxième couche de matériau rigide. Mais ces autres structures ne permettent pas de pallier aux problèmes cités précédemment.

L'invention vise à pallier tout ou partie des problèmes cités plus haut en proposant une structure innovante de cassette radiologique portable permettant de renforcer la rigidité de cette dernière, d'assurer une meilleure résistance contre la déformation et de protéger plus efficacement les éléments fragiles contenus dans la cassette radiologique portable.

A cet effet, l'invention a pour objet une cassette radiologique portable, selon la revendication 1, comprenant :
- un scintillateur apte à convertir un rayon X incident en un signal lumineux,
- une dalle photosensible apte à convertir le signal lumineux émis par le scintillateur en une charge électrique, le scintillateur et la dalle photosensible formant un panneau, le panneau ayant une face avant destinée à recevoir le rayon X incident et une face arrière opposée à la face avant,
- une carte électronique assurant la conversion de la charge électrique en une image digitale,
- un boîtier de protection mécanique, dans lequel sont disposés le panneau et la carte électronique, comportant une face supérieure et une face inférieure;

caractérisée en ce que la face supérieure du boîtier de protection mécanique comprend :
- une première couche de matériau rigide,
- une deuxième couche de matériau rigide, la deuxième couche de matériau rigide étant en contact avec la face avant du panneau,
- une couche de matériau alvéolaire disposée entre la première et la deuxième couche de matériau rigide.

Selon un aspect de l'invention, la couche de matériau alvéolaire est en matériau expansé.

Selon un aspect de l'invention, la couche de matériau alvéolaire comprend un empilement de tubes au moins partiellement creux s'étendant sensiblement perpendiculairement par rapport à la face avant du panneau.

Selon un aspect de l'invention, la couche de matériau alvéolaire comprend une multitude de billes.

Selon un aspect de l'invention, les billes sont creuses.

Selon un aspect de l'invention, la deuxième couche de matériau rigide est collée à la face avant du panneau.

Selon un aspect de l'invention, la couche de matériau alvéolaire est définie par une troisième épaisseur et la première et la deuxième couche de matériau rigide sont respectivement définies par une première et une deuxième épaisseur, la première et deuxième épaisseur étant inférieures à la troisième épaisseur de la couche de matériau alvéolaire.

Selon un aspect de l'invention, la couche de matériau alvéolaire est constituée d'un composite organique.

Selon un aspect de l'invention, la première et/ou la deuxième couche de matériau rigide est constituée d'aluminium et/ou de magnésium et/ou de composite de fibres organiques, de carbone ou minérales.

Selon un aspect de l'invention, la cassette radiologique portable comporte une couche de protection anti-rétrodiffusion disposée contre la face arrière du panneau, la couche de protection anti-rétrodiffusion étant préférentiellement constituée d'au moins un matériau de masse atomique élevée.

Selon un aspect de l'invention, la cassette radiologique portable comporte une couche d'isolation thermique, la couche d'isolation thermique étant positionnée entre la carte électronique et la face arrière du panneau.

L'invention sera mieux comprise et d'autres avantages apparaîtront à la lecture de la description détaillée d'un mode de réalisation donné à titre d'exemple, description illustrée par le dessin joint dans lequel :
[Fig.1] la figure 1 représente schématiquement une vue en coupe d'une structure connue de l'art antérieur de cassette numérique portable ;
[Fig.2] la figure 2 représente schématiquement une structure de cassette numérique portable selon l'invention ;
[Fig.3] la figure 3 représente schématiquement une vue éclatée d'une face supérieure d'un boîtier de la cassette numérique portable selon l'invention ;
[Fig.4] la figure 4 représente schématiquement une vue éclatée d'une face supérieure d'un boîtier de la cassette numérique portable selon une variante de l'invention.

Par souci de clarté, les mêmes éléments porteront les mêmes repères dans les différentes figures.

La figure 2 représente schématiquement une cassette numérique portable 10 selon l'invention. La cassette radiologique portable 10 comprend:
- un scintillateur 20 apte à convertir un rayon X incident en un signal lumineux,
- une dalle 30 photosensible apte à convertir le signal lumineux émis par le scintillateur 20 en une charge électrique. La dalle 30 photosensible est, à titre d'exemple indicatif, une matrice d'éléments photosensibles. Le scintillateur 20 et la dalle 30 photosensible forment un panneau 40 ayant une face avant 410 destinée à recevoir le rayon X incident et une face arrière 420 opposée à la face avant 410,
- une carte électronique 50 assurant la conversion de la charge électrique en une image digitale,
- un boîtier 60 de protection mécanique, dans lequel sont disposés le panneau 40 et la carte électronique 50, comportant une face supérieure 610 et une face inférieure 620.

La face supérieure 610 du boîtier 60 de protection mécanique comprend :
- une première couche 611 de matériau rigide, constituée d'aluminium et/ou de magnésium et/ou de composite de fibres organiques, de carbone ou minérales;
- une deuxième couche 612 de matériau rigide. La deuxième couche 612 de matériau rigide est en contact direct avec la face avant 410 du panneau 40. Plus précisément, le scintillateur 20 du panneau 40 repose contre la deuxième couche 612 de matériau rigide. Ainsi, la deuxième couche 612 de matériau rigide assure le rôle de structure rigide et permet donc le maintien rigide du panneau 40 qui est un élément fragile. Sans ce contact, une simple torsion du panneau pourrait impliquer une dégradation du panneau 40, ce qui n'est pas souhaitable. Préférentiellement, la deuxième couche 612 est obtenue dans le même matériau rigide que la première couche 611 mais peut être constituée d'un matériau différent de la première couche 611 ;
- une couche de matériau alvéolaire 613 disposée entre la première et la deuxième couche de matériau rigide 611 et 612. La couche de matériau alvéolaire 613 peut être en matériau expansé.

Cette structure d'empilement de la première couche 611, de la couche de matériau alvéolaire 613 et de la deuxième couche 612 de matériau rigide s'apparente à une structure dite « sandwich ». La couche de matériau alvéolaire 613 est ainsi en contact avec la première couche de matériau rigide 611 et avec la deuxième couche de matériau rigide 612 de sorte à remplir intégralement un espace dans le boîtier 60 de protection mécanique entre la première couche de matériau rigide 611 et la deuxième couche de matériau rigide 612. Cet empilement successif présente l'avantage d'assurer la rigidité globale, contre les chocs mais aussi en torsion, de l'ensemble tout en minimisant l'absorption des rayons X. En variante, la couche de matériau alvéolaire 613 peut être solidaire de la première couche de matériau rigide 611 et de la deuxième couche de matériau rigide 612. A titre d'exemple indicatif, cette solidarité peut se faire par l'intermédiaire d'un collage.

Grâce à cette nouvelle structure, l'embase formée dans la cassette radiologique portable selon l'état de l'art peut être supprimée permettant à la nouvelle cassette radiologique portable d'obtenir un gain de masse.

Avantageusement, la première couche 611 et la deuxième couche 612 de matériau rigide absorbent faiblement les rayons X tout comme la couche de matériau alvéolaire 613, assurant ainsi la bonne réception des rayons X pour le scintillateur 20 du panneau 40. De plus, la deuxième couche 612 de matériau rigide peut être collée à la face avant 410 du panneau 40 afin de fixer de manière parfaite le panneau 40 contre la deuxième couche 612 et assurer la bonne rigidité du panneau 40. Ainsi, tout type de collage permanent permettant de coller le panneau 40 à la deuxième couche 612 de matériau rigide peut être utilisé, comme par exemple, un adhésif double face, une colle ductile pouvant sécher ou toute autre liaison chimique faible, dite de Van der Waals.

En outre, la cassette radiologique portable 10 selon l'invention peut comporter une couche de protection anti-rétrodiffusion 90 disposée contre la face arrière 420 du panneau 40. Idéalement, la couche de protection anti-rétrodiffusion 90 est en contact direct avec la face arrière 420 du panneau 40. La couche de protection anti-rétrodiffusion 90 est préférentiellement constituée d'un ou d'au moins un matériau de masse atomique élevée ou d'une combinaison de matériaux dont les numéros atomiques sont judicieusement choisis et est destinée à limiter la rétrodiffusion de rayons X en direction du panneau 40 dans une direction sensiblement opposée à la direction d'incidence Z des rayons X pouvant nuire au bon fonctionnement du panneau 40 et donc à la cassette radiologique portable 10. La cassette radiologique portable 10 peut aussi comprendre une plaque de blindage électromagnétique 92 disposée de l'autre côté de la couche de protection anti-rétrodiffusion 90 et contre la couche de protection anti-rétrodiffusion 90 afin d'isoler le panneau 40 des éventuelles ondes électromagnétiques générées par la carte électronique 50.

La cassette radiologique portable 10 peut comporter une couche d'isolation thermique 94, la couche d'isolation thermique 94 est positionnée entre la carte électronique 50 et la face arrière 420 du panneau 40 afin d'isoler le panneau de la chaleur générée par la carte électronique 50.

Enfin, la cassette radiologique portable 10 peut comprendre une source d'énergie (non représentée) de la carte électronique 50.

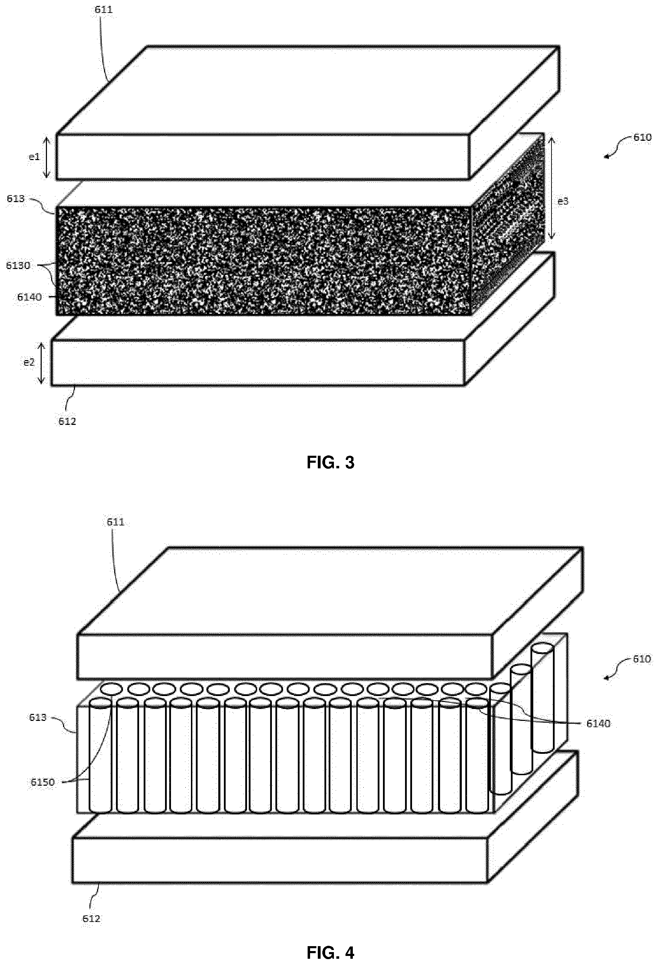

La figure 3 représente une vue éclatée de la face supérieure 610 du boîtier 60 de la cassette numérique portable 10. Comme dit précédemment, la face supérieure 610 du boîtier 60 de la cassette numérique portable 10 est définie par l'empilement successif de la première couche 611, de la couche de matériau alvéolaire 613 et de la deuxième couche 612 de matériau rigide.

Ainsi, la première couche 611 est définie par une première épaisseur e1, la deuxième couche 612 de matériau rigide est définie par une deuxième épaisseur e2 et la couche de matériau alvéolaire 613 est définie par une troisième épaisseur e3. Selon un aspect de l'invention, la première épaisseur e1 et la deuxième épaisseur e2 sont identiques. Ainsi, et à titre d'exemple, la première épaisseur e1 et la deuxième épaisseur e2 peuvent être comprise entre une épaisseur minimale d'environ 0,2 millimètre et une épaisseur maximale de 0,7 millimètre. Néanmoins, une structure dissymétrique peut aussi être envisagée. Ainsi, la première épaisseur e1 de la première couche 611 peut être différente de la deuxième épaisseur e2 de la deuxième couche 612. A titre d'exemple, la première épaisseur e1 peut être comprise entre 0,3 millimètre et 1,5 millimètre et la deuxième épaisseur e2 peut être comprise entre 0,3 millimètre et 1 millimètre. Préférentiellement, dans le cas d'une structure dissymétrique entre la première épaisseur e1 et la deuxième épaisseur e2, la première épaisseur e1 est supérieure à la deuxième épaisseur e2. En effet, pour la première couche 611, pouvant être comparée à la peau externe du boîtier 60, augmenter son épaisseur, c'est-à-dire augmenter l'épaisseur e1, permet d'augmenter l'épaisseur de la peau externe du boîtier 60 et permet donc d'augmenter la résistance du boîtier 60 contre les chocs et déformations ayant pour origine le milieu extérieur.

La troisième épaisseur e3 de la couche de matériau alvéolaire 613 est très supérieure à la première épaisseur e1 et à la deuxième épaisseur e2. Plus précisément, la troisième épaisseur e3 peut être par exemple comprise entre 2 millimètres et 4 millimètres. Ainsi, il est possible d'établir un ratio de grandeur entre la troisième épaisseur e3 et la somme de la première épaisseur e1 et de la deuxième épaisseur e2, et ce ratio de grandeur peut varier entre deux et huit selon les grandeurs des première, deuxième et troisième épaisseurs e1, e2 et e3.

Ainsi, ce faible dimensionnement de la première épaisseur e1 et de la deuxième épaisseur e2, très inférieures à la troisième épaisseur de la couche de matériau alvéolaire, additionné au fait que la couche de matériau alvéolaire 613 est peu absorbante du point de vue des rayons X, ne dégrade pas la qualité de l'image produite.

En effet, la couche de matériau alvéolaire 613 est constituée d'un composite organique qui absorbe peu ou pas les rayons X. Plus précisément, la couche de matériau alvéolaire 613 comprend, dans un premier mode de réalisation, une multitude de billes 6130. Ces billes 6130 semi rigides remplissent la troisième épaisseur e3 dans son intégralité. En outre, les billes 6130 ayant une forme ronde ou ovale et la couche de matériau alvéolaire 613 ayant une forme parallélépipédique, des espaces vides 6140 entre les billes 6130 apparaissent de manière homogène. Ainsi, lors d'un choc ou d'un effort induisant la déformation de la couche de matériau alvéolaire 613, les billes 6130 sont comprimées les unes contre les autres, diminuant ainsi les espaces vides 6140. En outre, les billes 6130 étant semi-rigides, elles peuvent aussi se déformer en cas de choc ou d'effort extrême appliqué sur la couche de matériau alvéolaire 613.

De cette manière, la couche de matériau alvéolaire 613 reste une couche rigide et non une mousse malléable tout en gardant la capacité d'être déformable à souhait afin d'encaisser la déformation liée à un choc ou à un effort à la place du panneau 40.

De surcroît et afin d'augmenter la capacité des billes 6130 à se déformer, les billes 6130 peuvent être des billes creuses.

Néanmoins, dans un deuxième mode de réalisation, la couche de matériau alvéolaire 613 peut comprendre, à la place des billes 6130, un empilement de tubes 6150 au moins partiellement creux s'étendant sensiblement perpendiculairement par rapport à la face avant 411 du panneau 40, comme représenté en figure 4. A l'image d'une structure interne de bambou, les tubes 6150 sont compartimentés par des noeuds, de telle sorte qu'ils peuvent être considérés comme un ensemble de tubulures séparées par des diaphragmes. Les tubes 6150, ainsi empilés dans la couche de matériau alvéolaire 613, sont en contact direct les uns contre les autres.

Les tubes 6150 peuvent être de section ovale, carrée ou rectangulaire mais sont préférentiellement de section hexagonale. Les tubes s'étendent de façon sensiblement parallèle à la direction d'incidence Z des rayons X dans la troisième épaisseur e3 de la couche de matériau alvéolaire 613. De cette manière, il existe aussi des espaces vides 6140 entre les tubes 6150 permettant à la couche de matériau alvéolaire 613 de facilement se déformer. En outre, les tubes 6150 peuvent aussi être déformables augmentant encore la capacité de la couche de matériau alvéolaire 613 à se déformer en cas de choc ou d'effort induisant une déformation de la couche de matériau alvéolaire 613.

En outre, dans un autre mode de réalisation privilégié, il peut être envisagé d'utiliser une couche de matériau alvéolaire 613 comprenant une mousse rigide définie comme une succession de matière déformable comme un matériau alvéolaire et de cavités macroscopiquement homogènes, cette mousse ayant une forme macroscopique matricielle comme une forme en nid d'abeille présentant alors une homogénéité macroscopique. Dans le cas contraire, la présence d'espaces vides de manière hétérogène dans la couche de matériau alvéolaire 613 peut induire des ombres portées sur l'image produite et donc dégrader la qualité de l'image produite.

De plus, il peut aussi être envisagé d'utiliser une structure expansée au sein de la couche de matériau alvéolaire 613 afin d'augmenter la capacité de cette dernière à se déformer.

Ainsi, la face supérieure 610 du boîtier 60 de la cassette numérique portable 10, qui est définie par l'empilement successif de la première couche 611, de la couche de matériau alvéolaire 613 et de la deuxième couche 612 de matériau rigide, présente une rigidité trois à dix fois supérieure à la rigidité du boîtier 6 cumulée à la rigidité de l'embase formée à l'intérieure du boîtier 6 selon l'état de l'art représenté en figure 1. De plus, la déformation de la face supérieure 610 du boîtier 60 lors d'une chute ou d'une flexion est réduite dans les mêmes proportions. Le panneau 40, collé sur cette face supérieure 610 par l'intermédiaire de la deuxième couche 612, est quasiment indéformable et ne subit ainsi plus de déformations susceptibles de l'endommager. Par ailleurs, cette structure en « sandwich » de l'empilement successif de la première couche 611, de la couche de matériau alvéolaire 613 et de la deuxième couche 612 de matériau rigide permet de limiter l'épaisseur de la face supérieure 610 à une épaisseur équivalente à celle communément utilisée dans la construction des cassettes radiologiques portables 1, respectant ainsi la norme ISO 4090, et ne dégrade pas la qualité de l'image produite, la couche de matériau alvéolaire 613 présentant une absorption des rayons X négligeable.

## Revendications

1. Cassette radiologique portable (10) comprenant :
- un scintillateur (20) apte à convertir un rayon X incident en un signal lumineux,
- une dalle (30) photosensible apte à convertir le signal lumineux émis par le scintillateur (20) en une charge électrique, le scintillateur (20) et la dalle (30) photosensible formant un panneau (40), le panneau (40) ayant une face avant (410) destinée à recevoir le rayon X incident et une face arrière (420) opposée à la face avant (410),
- une carte électronique (50) assurant la conversion de la charge électrique en une image digitale,
- un boîtier (60) de protection mécanique, dans lequel sont disposés le panneau (40) et la carte électronique (50), comportant une face supérieure (610) et une face inférieure (620);
la face supérieure (610) du boîtier (60) de protection mécanique comprenant :
- une première couche (611) de matériau rigide,
- une deuxième couche (612) de matériau rigide, la deuxième couche (612) de matériau rigide étant en contact avec la face avant (410) du panneau (40),
- une couche de matériau alvéolaire (613) disposée entre la première (611) et la deuxième (612) couche de matériau rigide, la cassette radiologique portable (10) étant **caractérisée en ce que** la couche de matériau alvéolaire (613) est en contact avec la première couche de matériau rigide (611) et avec la deuxième couche de matériau rigide (612).

2. Cassette radiologique portable (10) selon la revendication 1, dans laquelle la couche de matériau alvéolaire (613) est en matériau expansé.

3. Cassette radiologique portable (10) selon la revendication 1, dans laquelle la couche de matériau alvéolaire (613) comprend un empilement de tubes (6150) au moins partiellement creux s'étendant sensiblement perpendiculairement par rapport à la face avant (410) du panneau (40).

4. Cassette radiologique portable (10) selon la revendication 1, dans laquelle la couche de matériau alvéolaire (613) comprend une multitude de billes (6130).

5. Cassette radiologique portable (10) selon la revendication 4, dans laquelle les billes (6130) sont creuses.

6. Cassette radiologique portable (10) selon l'une des revendications 1 à 5, dans laquelle la deuxième couche (612) de matériau rigide est collée à la face avant (410) du panneau (40).

7. Cassette radiologique portable selon l'une des revendications 1 à 6, dans laquelle la couche de matériau alvéolaire (613) est définie par une troisième épaisseur (e3) et la première (611) et la deuxième (612) couche de matériau rigide sont respectivement définies par une première et une deuxième épaisseur (e1, e2), la première et deuxième épaisseur (e1, e2) étant inférieures à la troisième épaisseur (e3) de la couche de matériau alvéolaire (613).

8. Cassette radiologique portable (10) selon l'une des revendications 1 à 7, dans laquelle la couche de matériau alvéolaire (613) est constituée d'un composite organique.

9. Cassette radiologique portable (10) selon l'une des revendications 1 à 8, dans laquelle la première (611) et/ou la deuxième (612) couche de matériau rigide est constituée d'aluminium et/ou de magnésium et/ou de composite de fibres organiques, de carbone ou minérales.

10. Cassette radiologique portable (10) selon l'une des revendications 1 à 9, comportant une couche de protection anti-rétrodiffusion (90) disposée contre la face arrière (420) du panneau (40), la couche de protection anti-rétrodiffusion (90) étant préférentiellement constituée d'au moins un matériau de masse atomique élevée.

11. Cassette radiologique portable selon l'une des revendications 1 à 10, comportant une couche d'isolation thermique (94), la couche d'isolation thermique (94) étant positionnée entre la carte électronique (50) et la face arrière (420) du panneau (40).

## Patentansprüche

1. Tragbare radiologische Kassette (10), die Folgendes umfasst:
- einen Szintillator (20), der geeignet ist, um einen einfallenden Röntgenstrahl in ein Lichtsignal umzuwandeln,
- eine lichtempfindliche Platte (30), die geeignet ist, um das vom Szintillator (20) emittierte Lichtsignal in eine elektrische Ladung umzuwandeln, wobei der Szintillator (20) und die lichtempfindliche Platte (30) eine Tafel (40) bilden, wobei die Tafel (40) eine zum Empfangen des einfallenden Röntgenstrahls bestimmte Vorderseite (410) und eine der Vorderseite (410) gegenüberliegende Rückseite (420) aufweist,
- eine Leiterplatte (50), die die Umwandlung der elektrischen Ladung in ein digitales Bild gewährleistet,
- ein mechanisches Schutzgehäuse (60), in dem die Tafel (40) und die Leiterplatte (50) angeordnet sind, mit einer Oberseite (610) und einer Unterseite (620);
wobei die Oberseite (610) des mechanischen Schutzgehäuses (60) Folgendes umfasst:
- eine erste Schicht (611) aus steifem Material,
- eine zweite Schicht (612) aus steifem Material, wobei die zweite Schicht (612) aus steifem Material in Kontakt mit der Vorderseite (410) der Tafel (40) steht,
- eine Schicht aus zellulärem Material (613), die zwischen der ersten (611) und der zweiten (612) Schicht aus steifem Material angeordnet ist, wobei die tragbare radiologische Kassette (10) **dadurch gekennzeichnet ist, dass** die Schicht aus zellulärem Material (613) in Kontakt mit der ersten Schicht aus steifem Material (611) und mit der zweiten Schicht aus steifem Material (612) steht.

2. Tragbare radiologische Kassette (10) nach Anspruch 1, wobei die Schicht aus zellulärem Material (613) aus expandiertem Material besteht.

3. Tragbare radiologische Kassette (10) nach Anspruch 1, wobei die Schicht aus zellulärem Material (613) einen Stapel von mindestens teilweise hohlen Rohren (6150) umfasst, die sich im Wesentlichen senkrecht im Verhältnis zur Vorderseite (410) der Tafel (40) erstrecken.

4. Tragbare radiologische Kassette (10) nach Anspruch 1, wobei die Schicht aus zellulärem Material (613) eine Vielzahl von Perlen (6130) umfasst.

5. Tragbare radiologische Kassette (10) nach Anspruch 4, wobei die Perlen (6130) hohl sind.

6. Tragbare radiologische Kassette (10) nach einem der Ansprüche 1 bis 5, wobei die zweite Schicht (612) aus steifem Material mit der Vorderseite (410) der Tafel (40) verklebt ist.

7. Tragbare radiologische Kassette nach einem der Ansprüche 1 bis 6, wobei die Schicht aus zellulärem Material (613) durch eine dritte Dicke (e3) definiert ist und die erste (611) und die zweite (612) Schicht aus steifem Material jeweils durch eine erste und eine zweite Dicke (e1, e2) definiert sind, wobei die erste und die zweite Dicke (e1, e2) kleiner als die dritte Dicke (e3) der Schicht aus zellulärem Material (613) sind.

8. Tragbare radiologische Kassette (10) nach einem der Ansprüche 1 bis 7, wobei die Schicht aus zellulärem Material (613) aus einem organischen Verbundstoff besteht.

9. Tragbare radiologische Kassette (10) nach einem der Ansprüche 1 bis 8, wobei die erste (611) und/oder die zweite (612) Schicht aus steifem Material aus Aluminium und/oder Magnesium und/oder einem organischen Kohlenstoff- oder mineralischen Faserverbundstoff besteht.

10. Tragbare radiologische Kassette (10) nach einem der Ansprüche 1 bis 9, die eine an der Rückseite (420) der Tafel (40) angeordnete Anti-Rückstreu-Schutzschicht (90) umfasst, wobei die Anti-Rückstreu-Schutzschicht (90) vorzugsweise aus mindestens einem Material mit hoher Atommasse besteht.

11. Tragbare radiologische Kassette nach einem der Ansprüche 1 bis 10, die eine Wärmeisolationsschicht (94) umfasst, wobei die Wärmeisolationsschicht (94) zwischen der Leiterplatte (50) und der Rückseite (420) der Tafel (40) positioniert ist.

## Claims

1. A portable radiological cassette (10) comprising:
- a scintillator (20) capable of converting an incident x-ray into a light signal,
- a photosensitive slab (30) capable of converting the light signal emitted by the scintillator (20) into an electrical charge, the scintillator (20) and the photosensitive slab (30) forming a panel (40), the panel (40) having a front face (410) intended to receive the incident x-ray and a rear face (420) opposite the front face (410),
- an electronic circuit board (50) ensuring the conversion of the electrical charge into a digital image,
- a mechanical protection housing (60), in which the panel (40) and the electronic circuit board (50) are disposed, having a top face (610) and a bottom face (620);
the top face (610) of the mechanical protection housing (60) comprising:
- a first layer (611) of rigid material,
- a second layer (612) of rigid material, the second layer (612) of rigid material being in contact with the front face (410) of the panel (40),
- a layer of cellular material (613) disposed between the first (611) and the second (612) layer of rigid material, the portable radiological cassette (10) being **characterised in that** the layer of cellular material (613) is in contact with the first layer of rigid material (611) and with the second layer of rigid material (612).

2. The portable radiological cassette (10) according to claim 1, wherein the layer of cellular material (613) is made of expanded material.

3. The portable radiological cassette (10) according to claim 1, wherein the layer of cellular material (613) comprises a stack of at least partially hollow tubes (6150) extending substantially perpendicular with respect to the front face (410) of the panel (40).

4. Portable radiological cassette (10) according to claim 1, wherein the layer of cellular material (613) comprises a multitude of beads (6130).

5. The portable radiological cassette (10) according to claim 4, wherein the beads (6130) are hollow.

6. The portable radiological cassette (10) according to one of claims 1 to 5, wherein the second layer (612) of rigid material is glued to the front face (410) of the panel (40).

7. The portable radiological cassette according to one of claims 1 to 6, wherein the layer of cellular material (613) is defined by a third thickness (e3) and the first (611) and the second (612) layer of rigid material are respectively defined by a first thickness and a second thickness (e1, e2), the first thickness and second thickness (e1, e2) being smaller than the third thickness (e3) of the layer of cellular material (613).

8. The portable radiological cassette (10) according to one of claims 1 to 7, wherein the layer of cellular material (613) is composed of an organic composite.

9. The portable radiological cassette (10) according to one of claims 1 to 8, wherein the first (611) and/or the second (612) layer of rigid material is composed of aluminium and/or of magnesium and/or of carbon or mineral organic fibre composite.

10. The portable radiological cassette (10) according to one of claims 1 to 9, comprising an anti-backscatter protection layer (90) disposed against the rear face (420) of the panel (40), the anti-backscatter protection layer (90) being preferably composed of at least one material of high atomic mass.

11. The portable radiological cassette according to one of claims 1 to 10, having a thermal insulation layer (94), the thermal insulation layer (94) being positioned between the electronic circuit board (50) and the rear face (420) of the panel (40).
